# EUROPÄISCHE PATENTANMELDUNG

(11) **EP 0 524 584 A2**
(43) Veröffentlichungstag der Anmeldung: **27.01.1993**
(21) Anmeldenummer: 92112385.7
(22) Anmeldetag: 20.07.1992
(51) Int. Cl.: C07K 15/06, C07K 3/02, G01N 33/53, G01N 33/68

(54) **Verfahren zur Herstellung hochaffiner anti-Hapten-Antikörper und Mittel zur Bestimmung von Haptenen**

(30) Priorität: 26.07.1991 DE 4124853
(71) Anmelder: MERCK PATENT GmbH, D-64271 Darmstadt (DE)
(72) Erfinder: Stenzel, Roswitha, Dr., W-6940 Weinheim (DE); Reckmann, Bernd, Dr., W-6104 Seeheim-Jugenheim (DE); Würzburg, Uwe, Dr., W-6115 Münster (DE); Heubner, Arnulf, Dr., W-6500 Mainz (DE); Schönefeld, Ulrich, Dr., W-6100 Darmstadt (DE); Linxweiler, Winfried, Dr., W-6101 Messel (DE)

(57) **Zusammenfassung**

Die Erfindung betrifft ein Verfahren zur Herstellung hochaffiner anti-Hapten-Antikörper und ein Mittel zur Bestimmung von Haptenen mit Hilfe dieser Antikörper.

Das Verfahren zur Herstellung dieser hochaffinen anti-Hapten-Antikörper ist dadurch gekennzeichnet, daß man in einer ersten Stufe ein Tier mehrmals mit einem Hapten-Protein-Konjugat immunisiert und in der zweiten Stufe das Tier mehrmals mit einem Gemisch aus bis zu drei Hapten-Protein-Konjugaten immunisiert, wobei die Konjugate das gleich Hapten, jedoch verschiedene Proteine enthalten.

## Beschreibung

Die Erfindung betrifft ein Verfahren zur Herstellung hochaffiner anti-Hapten-Antikörper und ein Mittel zur Bestimmung von Haptenen mit Hilfe dieser Antikörper.

Haptene sind niedrigmolekulare Substanzen ohne immunogene Wirkung, deren Bestimmung in der klinischen Diagnostik eine zunehmende Bedeutung erlangt hat. Die Haptene werden erst durch Bindung an größere Moleküle immunogen und können dann mit Hilfe von immunologischen Methoden bestimmt werden.

Ein bekanntes Hapten ist z. B. das sehr wirksame Herzglykosid Digoxin, das zur Behandlung von kongestiver Herzinsuffizienz und bei bestimmten Herzrhytbmusstörungen verwendet wird. Da es eine geringe therapeutische Breite hat und die Serumspiegel nicht vorhersehbar sind, ist es wichtig, die Konzentration im Serum zu bestimmen. Diese Bestimmung wird mit Hilfe von Immunoassays durchgeführt. Da die Digoxin-Serumkonzentration im Vergleich zu anderen Medikamenten, z. B. Antiepileptica, sehr niedrig ist, müssen die Immunoassays sehr empfindlich sein. Die hohe Sensitivität kann unter anderem dadurch erreicht werden, daß sehr affine Antikörper in Immunoassays verwendet werden.

Die Herstellung von hochaffinen Antikörpern gegen Haptene ist mit den zur Zeit bekannten Immunisierungsmethoden nur sehr schwer möglich. Dabei wird das Hapten an ein Protein wie Rinderserumalbumin (BSA) oder Keyholelthemocyanin (KLH) kovalent gekoppelt. Die entstandene Substanz wird als Immunogen bezeichnet. Mit diesem Immunogen wird mit komplettem Freundschem Adjuvans eine Emulsion hergestellt, die in zwei- bis dreiwöchentlichem Abstand in bestimmte Tiere (Schafe, Ziegen, Mäuse, Kaninchen) injiziert wird. Nach 3 bis 6 Immunisierungen haben sich Antikörper gebildet. Min dieser Methode können mittelaffine Antkörper mit Affinitätskonstanten von K_{A} = 10⁷ - 10⁸ hergestellt werden; der Titer liegt zwischen 1/40 und 1/640. Hochaffine Antikörper (K_{A} = 10⁹ - 10¹²) werden nach dieser Methode nicht gebildet.

Der vorliegenden Erfindung liegt die Aufgabe zugrunde, ein Verfahren zur Herstellung hochaffiner anti-Hapten-Antikörper mit hohen Titern zur Verfügung zu stellen, die empfindliche Haptenbestimmungen ermöglichen.

Gegenstand der Erfindung ist ein Verfahren zur Herstellung hochaffiner anti-Hapten-Antikörper, das dadurch gekennzeichnet ist, daß man in einer ersten Stufe ein Tier mehrmals mit einem Hapten-Protein-Konjugat immunisiert und in der zweiten Stufe das Tier mehrmals mit einem Gemisch aus bis zu drei Hapten-Protein-Konjugaten immunisiert, wobei die Konjugate das gleiche Hapten, jedoch verschiedene Proteine enthalten.

Ein weiterer Gegenstand der Erfindung sind Mittel zur Bestimmung von Haptenen, die diese hochaffinen anti-Hapten-Antikörper enthalten.

Das Hapten-Protein-Konjugat (Immunogen) wird nach bekannten Verfahren hergestellt (Clin. Inv. 47, 1035-1042 (1968); A .Clin.Biochem. 20, 227-232 (1983)).

Überraschenderweise wurden mit der erfindungsgemäßen zweistufigen Immunisierungsmethode hochaffine anti-Hapten-Antikörper mit hohen Titern erhalten. Diese hochaffinen Antikörper erhält man auch, wenn man in der zweiten Immunisierungsstufe zwei anstelle von drei sich im Protein voneinander unterscheidende Konjugate verwendet oder wenn man die Immunisierung in der zweiten Stufe mit einem Konjugat durchführt, das sich von dem für die Immunisierung in der ersten Stufe verwendeten Konjugat durch das Protein unterscheidet.

Als Haptene im Hapten-Protein-Konjugat können in der zweiten Immunisierungsstufe auch Haptenderivate mit der gleichen immunogenen Determinante verwendet werden oder auch Konjugate, die sich im Haptenderivat und im Protein unterscheiden.

Zum Beispiel wurden Digoxin bzw. Succinyldigoxigenin an BSA, KLH und Pferde-IgG gekoppelt. Aus diesen drei Immunogenen wurde eine Mischung hergestellt. Die Tiere wurden in zwei Stufen immunisiert. In der ersten Stufe wurden sie mehrmals mit Digoxin-BSA oder Digoxin-KLH oder Digoxin-IgG immunisiert, in der zweiten Stufe wurden sie mehrmals mit der Immunogen-Mischung immunisiert. Nach der 3. bis 6. Immunisierung mit der Mischung kam es zu einem 30 bis 200%igen Anstieg des Hubes und einem starken Anstieg des Titers auf 1/5120 bis 1/10240. Ohne Änderung des Immunogens wären Titer und Affinität des Antikörpers konstant niedrig geblieben.

Das gleiche Immunisierungsverfahren wurde auch für andere Haptene wie Phenytoin, Phenobarbital, Carbamazepin, Primidon, Valproinsäure, Ethosuximid, Theophyllin, Gentamycin, Tobramycin, Amikacin, Netilmycin, Vancomycin, Kanamycin, Streptomycin, Dibekacin, Methotrexat, Cyclosporin, Digitoxin, Disopyramid, Flecainid, Procainamid, N-Acetylprocainamid, Lidocain, Chinidin, Cortisol, Testosteron, Progesteron, Östriol, Tetrajodthyronin, Trijodthyronin, Amphetamin, Metamphetamin, Opiate, Morphin, Benzodiazepin, Kokain, Barbiturat, Phencyclidin, Methadon, Cannabis, mit analogem Erfolg und guter Reproduzierbarkeit angewendet. Aufgrund des erfindungsgemäßen Verfahrens konnten zur Bestimmung von Haptenen sehr sensitive Immunoassays entwickelt werden.

Die Bestimmung der Antikörper-Titer erfolgt mit Hilfe des Fluoreszenzpolarisationsimmunoassays (FPIA). Dabei wird das Hapten mit einem Fluoreszenzfarbstoff markiert. Wenn dieses Reagenz (Tracer) mit polarisiertem Licht angeregt wird, ist das emittierte Licht dann polarisiert, wenn es sich um große Moleküle handelt, die sich langsam bewegen. Beim EPIA ist das vom Tracer emittierte Licht jedoch nicht polarisiert, weil der Tracer ein kleines Molekulargewicht hat. Der gebundene Tracer strahlt polarisiertes Licht ab, da durch die Bindung an den Antikörper ein großer Komplex entsteht. Dieser Immunoassay kann deshalb homogen durchgeführt werden, d. h. der freie Tracer muß vom gebundenen Tracer nicht getrennt werden. Zur Bestimmung der Haptenkonzentration wird der Test kompetitiv durchgeführt, d. h. Hapten und Tracer konkurrieren um die Bindung an den Antikörper. Je höher die Konzentration des Haptens ist, desto weniger Tracer wird gebunden. Das Meßsignal ist also umgekehrt proportional zur Haptenkonzentration.

### Beispiel 1

### 1. Immunogenherstellung

### 1.1 Kopplung von Succinyldigoxigenin an BSA

50 mg BSA werden in 25 ml Wasser gelöst (pH 5,5). Dazu werden 30 mg EDC (1-Ethyl-3-(3-dimethylaminopropyl)carbodiimid Hydrochlorid) gegeben. 20 mg Succinyldigoxigenin werden in 3,5 ml Dimethylformamid gelöst und tropfenweise unter Rühren zur obigen Lösung gegeben. Der pH-Wert wird auf 5,5 eingestellt. Nach 10 Minuten werden erneut 10 mg EDC hinzugefügt. Die Reaktionslösung wird 18 Stunden bei 25 °C gerührt und anschließend 5 Tage gegen fließendes Wasser dialysiert.

### 1.2 Kopplung von Succinyldigoxigenin an KLH

Die Reaktion erfolgt analog Beispiel 1.1, wobei anstelle von 50 mg BSA 20 mg KLH engesetzt werden.

### 1.3 Kopplung von Succinyldigoxigenin an IgG

Die Reaktion erfolgt analog Beispiel 1.1, wobei anstelle von 50 mg BSA 25 mg IgG eingesetzt werden.

### 1.4 Kopplung von Digoxin an BSA

210 mg (0,27 mM) Digoxin werden in 10 ml Ethanol gelöst. Zu dieser Lösung werden tropfenweise 10 ml einer 0,1 M Natriummetaperjodatlösung gegeben. Nach 25 Minuten werden 0,3 ml einer 1 M Ethylenglycollösung hinzugefügt.

In einem anderen Gefäß werden 280 mg BSA in 8 ml Wasser gelöst, das mit 5%iger Kaliumcarbonatlösung auf einen pH-Wert von 9,5 eingestellt wird. Das aktivierte Digoxin wird tropfenweise zu dieser BSA-Lösung hinzugegeben. Nach 45 Minuten wird der pH-Wert auf 9,3 eingestellt und die Lösung mit einer Natriumborhydridlösung (0,15 g/10 ml Wasser) versetzt. Die Lösung wird 3 Stunden bei 25 °C gerührt. Mit einer 1 M Ameisensäure wird der pH-Wert auf 6,5 eingestellt; die Lösungwird eine weitere Stunde inkubiert. Der pH-Wert wird anschließend mit einer 1 M Ammoniaklösung auf 8,5 eingestellt und die Lösung 5 Tage gegen fließendes Wasser dialysiert.

### 1.5. Kopplung von Digoxin an KLH

Die Kopplung erfolgt analog Beispiel 1.4, wobei anstelle von 280 mg BSA 100 mg KLH eingesetzt werden.

### 1.6 Kopplung von Digoxin an IgG

Die Kopplung erfolgt analog Beispiel 1.4, wobei anstelle von 280 mg BSA 150 mg IgG eingesetzt werden.

### 1.7 Kopplung von Digoxigeninbisdigitoxosid an BSA

Die Reaktion erfolgt analog Beispiel 1.4, wobei anstelle von 210 mg (0,27 mM) Digoxin 185 mg (0,27 mM) Digoxigeninbisdigitoxosid eingesetzt werden.

### 1.8 Kopplung von Lanatosid an KLH

Die Reaktion erfolgt analog Beispiel 1.5, wobei anstelle von 210 mg (0,27 mM) Digoxin 266 mg Lanatosid eingesetzt werden.

### 1.9 Kopplung von 4-Aminophenylprimidon an BSA

Zu einer Lösung von 4-Aminophenylprimidon in 2 ml 0,1 M Salzsäure werden 20 mg festes Natriumnitrit gegeben und 10 Minuten bei 0 °C gerührt. Die überschüssige salpetrige Säure wird durch Zugabe von 20 mg Harnstoff zerstört. Der pH-Wert der Lösung wird mit einer 1 M Natriumbicarbonatlösung auf 8 - 9 eingestellt. Das diazotierte Aminoprimidon wird anschließend tropfenweise und unter Rühren in eine Lösung von 2 ml BSA (30 mg/ml) gegeben. Die Lösung wird 30 Minuten bei 0 °C gerührt und dann 2 Tage gegen fließendes Wasser dialysiert.

### 1.10 Kopplung von Trijodthyronin (T₃) an BSA

50 mg BSA werden in 25 ml Wasser gelöst (pH 5,5) und 30 mg EDC hinzugegeben. 20 mg Trijodthyronin (freie Säure) werden in 2 ml Dimethylformamid gelöst und tropfenweise unter Rühren zur obigen Lösung gegeben. Der pH-Wert wird auf 5,5 eingestellt. Nach 10 Minuten werden nochmal 10 mg EDC hinzugefügt. Die Reaktionslösung wird 18 Stunden bei 25 °C gerührt und dann 5 Tage gegen fließendes Wasser dialysiert.

### 1.11 Kopplung von Amikacin an Thyroglobulin

100 mg Thyroglobulin werden in 5 ml Wasser gelöst und mit 5 ml Amikacinlösung (8 mg/ml) gemischt.

334 mg EDC werden in 10 ml Wasser gelöst und tropfenweise unter kräftigem Rühren innerhalb von 30 Minuten zugegeben. Die Lösung wird eine Stunde bei Raumtemperatur und 24 Stunden bei 4 °C inkubiert. Das Immunogen wird durch Säulenchromatographie mit einer Sephadex^{R} G25 Säule gereinigt.

### 1.12 Kopplung von Cortisol-3-CMO an BSA

130 mg Cortisol-3-CMO werden in 23 ml Dioxan gelöst. 2,5 ml Triethylamin und 2,5 ml Butylchloroformiat werden zu der Lösung gegeben und 30 Minuten bei 20 °C gerührt. 100 mg BSA werden in 10 ml Wasser gelöst und der pH-Wert wird mit 0,1 M NaOH auf 9 eingestellt.

Die Cortisollösung wird zu der BSA-Lösung gegeben und nach 5 Minuten wird der pH-Wert auf 8,5 eingestellt. Der Ansatz wird 4 Stunden bei 4 °C gerührt und dann 3 Tage gegen Wasser dialysiert.
- 1.13: Herstellung der Immunogengemische
- 1.13.1: 5 mg Succinyldigoxigenin-BSA, 5 mg Succinyldigoxigenin-KLH und 5 mg Succinyldigoxigenin-IgG werden 1 + 1 + 1 gemischt.
- 1.13.2: 10 mg Amikacin-IgG und 50 mg Amikacin-KLH werden 1 + 5 gemischt.
- 1.13.3: 1 mg Succinyldigoxigenin-BSA, 3 mg Lanatosid-KLH und 7 mg Digoxin-IgG werden 1 + 3 + 7 gemischt.
- 1.13.4: 10 mg Trijodthyronin-BSA, 5 mg Trijodthyronin-KLH und 20 mg Trijodthyronin-IgG werden 1 + 0,5 + 2 gemischt.

### Beispiel 2

### Bestimmung der Antikörper-Titer

Zur Bestimmung des Antikörper-Titers wird eine Antikörper-Titration nach folgendem Pipettierschema durchgeführt:
50 µl Antikörper-Verdünnung
900 µl Puffer

### Leerwert-Messung

50 µl Tracer
5 Minuten Inkubation bei 37 °C
Es werden 8 unterschiedliche Antikörper-Verdünnungen eingesetzt.

Der gleiche Versuch wird gemacht, indem gleichzeitig eine Verdrängungsreaktion mit der höchsten Konzentration an Hapten, die in der Standardkurve verwendet wird, durchgeführt wird.
50 µl Antikörper-Verdünnung
50 µl Standard
850 µl Puffer

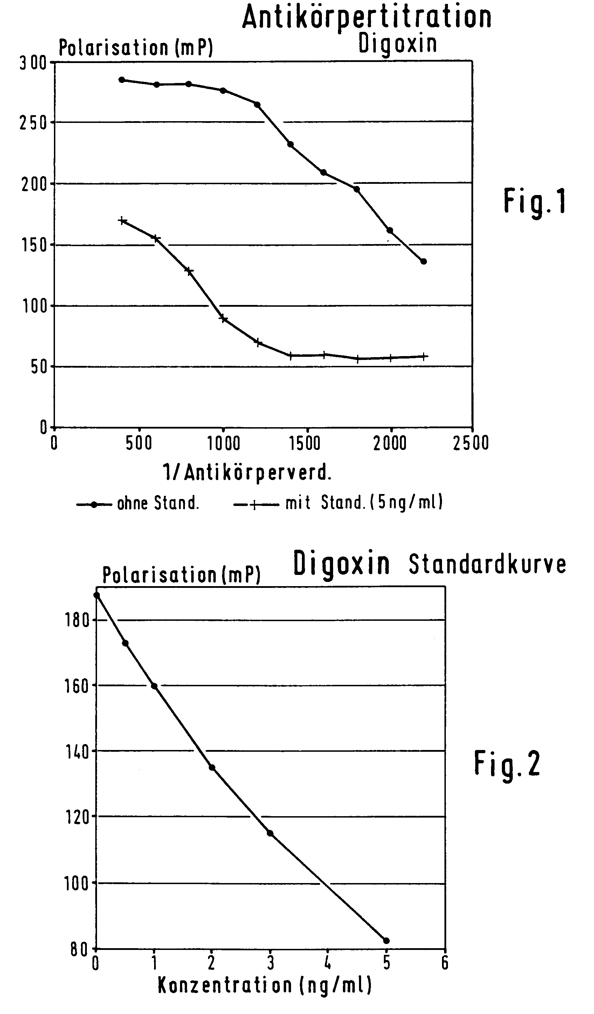

### Leerwert-Messung 50 µl Tracer 5 Minuten Inkubation bei 37 °C Abbildung 1 zeigt das Ergebnis einer Antikörper-Titration für Digoxin. Der Titer ist die Antikörper-Verdünnung, bei der die Differenz des Meßsignals zwischen dem Meßwert ohne Standard und dem Meßwert mit Standard (Hub) am größten ist. Der Titer in Abbildung 1 ist 1/1280.

### Beispiel 3

### Messung einer Standardkurve

Zur Erstellung einer Standardkurve wird eine bestimmte Antikörper-Menge und 6 verschiedene Standardkonzentrationen eingesetzt. Das Pipettierschema entspricht dem in Beispiel 2 beschriebenen Schema mit Standard. Abbildung 2 zeigt eine Standardkurve für Digoxin.

### Beispiel 4

### Einstufige Immunisierung

0,8 mg Succinyldigoxigenin-BSA werden mit einem Adjuvans (Komplettes oder inkomplettes Freundsches Adjuvans, Aluminiumhydroxid, Wasser-Öl Bmulsion oder Muramyldipeptid) emulgiert. Mit dieser Emulsion wird das Tier zweimal im Abstand von 21 Tagen immunisiert. Danach wird es 4 - 12mal mit einer Emulsion aus 0,8 mg Succinyldigoxigenin-BSA mit inkomplettem Freundschen Adjuvans im Abstand von 21 Tagen immunisiert.

Die Tabelle 1 zeigt den Titerverlauf bei einem Schaf, das nach diesem Verfahren immunisiert worden ist.

**Tabelle 1**

| Boost | Hub | Titer |
|---|---|---|
| 4 | 29 | 80 |
| 5 | 42 | 80 |
| 6 | 29 | 160 |
| 7 | 40 | 320 |
| 8 | 38 | 160 |
| 9 | 40 | 160 |
| 10 | 38 | 160 |
| 11 | 36 | 160 |
| 12 | 40 | 160 |
| 13 | 42 | 160 |
| 14 | 39 | 160 |

Nach dem 5. Boost ist der Hub konstant niedrig zwischen 30 - 40 mP und der Titer ist konstant niedrig bei 1/160.

### Beispiel 5

### Immunisierung in zwei Stufen

### 5.1

Erste Stufe: Immunisierung mit Succinyldigoxigenin-BSA, zweite Stufe: Immunisierung mit einem Gemisch aus Succinyldigoxigenin-BSA, Succinyldigoxigenin-KLH und Succinyldigoxigenin-IgG im Schaf.

0,8 mg eines nach 1.13.1 hergestellten Immunogen-Gemisches werden mit komplettem Freundschen Adjuvans emulgiert. Nachdem das Tier, wie in Beispiel 4 beschrieben, 8mal immunisiert worden ist, wird es danach 2mal im Abstand von 21 Tagen mit 0,8 mg des Gemisches in komplettem Freundschen Adjuvans immunisiert. Dann wird es 3mal mit je 0,8 mg des Gemisches in inkomplettem Freundschen Adjuvans im Abstand von 21 Tagen immunisiert.

Der Titer wird nach Beispiel 2 bestimmt. Die Tabelle 2 zeigt das Ergebnis einer solchen Immunisierung. Nachdem der Hub nach der 8. Immunisierung mit Succinyldigoxigenin-BSA nahezu konstant bleibt, steigt er nach der 2. bis 5. Immunisierung mit dem Gemisch um das Doppelte an. Der Titer bleibt bis zur 10. Immunisierung konstant und steigt von der 11. bis 13. Immunisierung von 1/640 bis 1/5120.

**Tabelle 2**

| Boost | Hub | Titer | |
|---|---|---|---|
| 3 | 35 | 640 | 1. Stufe |
| 4 | 35 | 640 | |
| 5 | 37 | 640 | |
| 6 | 36 | 640 | |
| 7 | 38 | 640 | |
| 8 | 39 | 640 | |
| 9 | 39 | 640 | 2. Stufe |
| 10 | 40 | 640 | |
| 11 | 70 | 2560 | |
| 12 | 82 | 5120 | |
| 13 | 83 | 5120 | |

### 5.2 Erste Stufe: Immunisierung mit Succinyldigoxigenin-KLH, Zweite Stufe: Immunisierung mit Digoxin-BSA in der Maus

Succinyldigoxigenin-KLH wird mit komplettem Freundschen Adjuvans emulgiert. Das Tier wird fünfmal mit dieser Emulsion, die jeweils 0,2 mg des Immunogens enthält, immunisiert. Danach wird es fünfmal mit jeweils 0,2 mg Digoxin-BSA immunisiert. Der bis zum 6. Boost konstante Hub und Titer steigen nach der Immunisierung mit Digoxin-BSA langsam an, bis der Hub beim 9. Boost um das 2,5fache angestiegen ist und der Titer sich vervierfacht hat, wie die nachstehende Tabelle 3 zeigt.

**Tabelle 3**

| Boost | Hub | Titer | |
|---|---|---|---|
| 3 | 40 | 320 | 1. Stufe |
| 4 | 42 | 320 | |
| 5 | 38 | 320 | |
| 6 | 41 | 320 | 2. Stufe |
| 7 | 50 | 320 | |
| 8 | 84 | 640 | |
| 9 | 103 | 1280 | |
| 10 | 109 | 1280 | |

### 5.3 Erste Stufe: Immunisierung mit Amikacin-BSA, zweite Stufe: Immunisierung mit einem Gemisch aus Amikacin-KLH und Amikacin-IgG im Schaf

Das Schaf wurde fünfmal mit einer Losung aus inkomplettem Freundschen Adjuvans und Amikacin-BSA immunisiert (0,8 mg pro Boost). Dann wurde es fünfmal mit dem Immunogen-Gemisch (0,8 mg pro Boost) nach 1.13.2 immunisiert. Wie aus Tabelle 4 ersichtlich ist, bleiben Hub und Tier konstant bis zum 1. Boost mit dem Immunogengemisch. Nach 4 Boosts mit dem Immunogen-Gemisch ist der Hub um das vierfache und der Titer um das achtfache angestiegen.

**Tabelle 4**

| Boost | Hub | Titer | |
|---|---|---|---|
| 3 | 20 | 80 | 1. Stufe |
| 4 | 30 | 80 | |
| 5 | 32 | 80 | |
| 1 | 29 | 80 | 2. Stufe |
| 2 | 50 | 320 | |
| 3 | 100 | 320 | |
| 4 | 124 | 640 | |
| 5 | 126 | 640 | |

### 5.4 Erste Stufe: Immunisierung mit Primidon-BSA, zweite Stufe: Immunisierung mit Primidon-IgG im Kaninchen

Das Kaninchen wurde 5mal mit einer Lösung aus Aluminiumhydroxid und Primidon-BSA immunisiert. Dann wurde es 5mal mit Primidon-IgG immunisiert. Wie aus Tabelle 5 zu erkennen ist, steigen Hub und Titer nach der 2. Immunisierung mit Primidon-IgG langsam an. Bei der 4. Immunisierung mit Primidon-IgG hat sich der Hub verdreifacht und der Titer verachtfacht.

**Tabelle 5**

| Boost | Hub | Titer | |
|---|---|---|---|
| 3 | 48 | 40 | 1. Stufe |
| 4 | 50 | 40 | |
| 5 | 53 | 40 | |
| 1 | 60 | 40 | 2. Stufe |
| 2 | 64 | 40 | |
| 3 | 80 | 160 | |
| 4 | 141 | 320 | |
| 5 | 138 | 320 | |

### 5.5 Erste Stufe: Immunisierung mit Trijodthyronin-BSA, zweite Stufe: Immunisierung mit einem Gemisch aus Trijodthyronin-BSA, Trijodthyronin-KLH, Trijodthyronin-IgG in der Ziege.

Die Ziege wurde zunächst 5mal mit jeweils 0,8 mg Trijodthyronin-BSA immunisiert und dann 8mal mit dem Immunogengemisch nach 1.13.4. Durch die Immunisierung mit dem Immunogengemisch steigt der Hub um 50 % und der Titer um das 4fache an, wie die nachstehende Tabelle 6 zeigt.

**Tabelle 6**

| Boost | Hub | Titer | |
|---|---|---|---|
| 3 | 22 | 160 | 1. Stufe |
| 4 | 50 | 640 | |
| 5 | 55 | 640 | |
| 1 | 61 | 640 | 2. Stufe |
| 2 | 60 | 640 | |
| 3 | 62 | 640 | |
| 4 | 70 | 640 | |
| 5 | 75 | 1280 | |
| 6 | 83 | 2560 | |
| 7 | 94 | 2560 | |
| 8 | 96 | 2560 | |

## Patentansprüche

1. Verfahren zur Herstellung hochaffiner anti-Hapten-Antikörper, dadurch gekennzeichnet, daß man in einer ersten Stufe ein Tier mehrmals mit einem Hapten-Protein-Konjugat immunisiert und in der zweiten Stufe das Tier mehrmals mit einem Gemisch aus bis zu drei Hapten-Protein-Konjugaten immunisiert, wobei die Konjugate das gleiche Hapten, jedoch verschiedene Proteine enthalten.

2. Verfahren nach Anspruch 1, dadurch gekennzeichnet, daß man ein Gemisch aus zwei sich im Protein voneinander unterscheidenden Konjugaten verwendet.

3. Verfahren nach den Ansprüchen 1 und 2, dadurch gekennzeichnet, daß man die Immunisierung in der zweiten Stufe mit einem Konjugat durchführt, das sich von dem für die Immunisierung in der ersten Stufe verwendeten Konjugat durch das Protein unterscheidet.

4. Verfahren nach den Ansprüchen 1 bis 3, dadurch gekennzeichnet, daß man als Hapten im Hapten-Protein-Konjugat der zweiten Immunisierungsstufe Haptenderivate mit der gleichen immunogen Determinante verwendet.

5. Verfahren nach den Ansprüchen 1 bis 4, dadurch gekennzeichnet, daß man in der zweiten Immunisierungsstufe sich im Haptenderivat und im Protein unterscheidende Konjugate verwendet.

6. Mittel zur Bestimmung von Haptenen, enthaltend die nach dem Verfahren nach den Ansprüchen 1-5 hergestellten hochaffinen anti-Hapten-Antikörper.
